Europäisches Patentamt

European Patent Office

Office européen des brevets

(11) Publication number: **0 051 293**
**B1**

(12) **EUROPEAN PATENT SPECIFICATION**

(45) Date of publication of patent specification: **14.08.85**

(51) Int. Cl.⁴: **A 61 B 5/08,** G 01 F 1/66

(21) Application number: **81109303.8**

(22) Date of filing: **29.10.81**

(54) **Respiration flowmeter.**

(30) Priority: **31.10.80 JP 153261/80**
**31.10.80 JP 153264/80**

(43) Date of publication of application:
**12.05.82 Bulletin 82/19**

(45) Publication of the grant of the patent:
**14.08.85 Bulletin 85/33**

(84) Designated Contracting States:
**DE FR GB NL**

(56) References cited:
**US-A-3 237 453**
**US-A-3 329 017**
**US-A-3 918 304**
**US-A-3 922 525**
**US-A-4 052 896**
**US-A-4 095 457**

**IEEE TRANSACTIONS ON BIOMEDICAL
ENGINEERING; vol. BME-27, no. 10. October
1980 New York, US D.I. PLAUT et al.
"Ultrasonic measurement of respiratory flow"
pages 549-558**

(73) Proprietor: **Kabushiki Kaisha Toshiba
72, Horikawa-cho Saiwai-ku
Kawasaki-shi Kanagawa-ken 210 (JP)**

(72) Inventor: **Ogura, Ichiro
1221 Maeda-Heights 511 Maeda-cho
Totsuka-ku Yokohama-shi (JP)**
Inventor: **Itoh, Ayao
29-299, Nagahama Kanazawa-ku
Yokohama-shi (JP)**

(74) Representative: **Henkel, Feiler, Hänzel & Partner
Möhlstrasse 37
D-8000 München 80 (DE)**

(56) References cited:
**HEWLETT PACKARD JOURNAL vol. 30, no. 9,
September 1979, Palo Alto, US M.R. BLAIS et
al. "Automated pulmonary function
measurements", pages 20-24**

Courier Press, Leamington Spa, England.

## Description

The present invention relates to a respiration flowmeter comprising a conduit through which expiration and inspiration flow, the conduit having a pair of recesses formed respectively on opposite portions of the inner wall of the conduit along an axis inclined to the direction of the respiration gas flow; a pair of ultrasonic transducers disposed in the recesses, respectively, and having ultrasonic transmitting and receiving surfaces facing each other and perpendicular to the axis; switching means for simultaneously switching the operation modes of said ultrasonic transducers, from a drive mode to a receiving mode, and vice versa; digital counting means for receiving signals generated by the transducers upon reception of ultrasonic pulses and measuring the two propagating periods required by ultrasonic pulses radiated from each of said driven transducers to reach the receiving transducers and the difference between these propagating periods; judging means for judging the direction of the respiration gas flow; means for generating a signal for requesting calculation of a flow velocity of the respiration gas every time said transducers are driven; storing means for storing signals representing said flow direction, the propagation periods, and said propagation period difference, in response to said calculation request signal; and means for calculating and storing the flow rate of said respiration gas on the basis of the values stored in said storing means, said calculating and storing means including an arithmetic process for obtaining the flow velocity V by executing the following operation:

$$V = \frac{-2(2d+L)^2}{L \cos \theta} \cdot \frac{\Delta T}{(T1+T2)}$$

where d is the length of the ultrasonic transmission path in one of said recesses free from the effect of the flow velocity of said gas, L is the length of the ultrasonic transmission path in which the flow velocity of said gas is finite, θ is the angle between the respiration flow direction and the axis, T1 is the ultrasonic propagating period of the ultrasonic pulses from one to the other of the pair of ultrasonic transducers, T2 is the ultrasonic propagating period of the ultrasonic pulses radiated from the other to the one of the pair of the ultrasonic transducers, and ΔT is the difference time difference between the two propagating periods T1 and T2.

Document "IEEE Transactions on Boimedocal Engineering, BME-27, October 1980, pages 549—558 discloses a similar ultrasonic measurement of the respiratory flow in which, to compute the flow velocity, the sum of the downstream transit time and the upstream transit time is used. An ultrasonic transducer is disposed in a recess of a conduit. However, the length of this recess is not included in the length of the transducer separation which reduces the accuracy of the measurements, and an accurate measurement of the transit times requires the use of high frequency clocks so that a complex circuit for performing the calculations is needed.

Furthermore, US—A—3,237,453 and 3,329,017 disclose ultrasonic flowmeter apparatus which use so-called "sing around systems" in which two probes are reversed in their operation modes as transmitter and receiver of ultrasonic waves, respectively, by means of switching means, and a counter counts the difference between the frequencies of the pulse repetition in the cocurrent and the counter-current cases, thereby to measure the flow velocity of the fluid flowing in a vessel.

Finally, US—A—4,052,096 discloses an ultrasonic flowmeter which uses the technique of mixing two signals of different frequencies, thus obtaining sideband frequencies. These sideband frequencies are supplied to a transducer which causes ultrasonic waves to penetrate a fluid flow.

Recently, there has been recognized the necessity of monitoring a respiration function of a patient, inter alia a postoperative serious case. To effect this, it is essential to continuously monitor a flow velocity or flow rate of the respiration, as its basic approach. A first and important requirement for the inspiration flowmeter used for such end is not to inflict a physical and mental load on the patient. Particularly, the respiration flowmeter structured to give a difficulty in breathing, is improper. Conventional flowmeters such as differential-pressure type flowmeters, hot-wire type flowmeters or turbine type flowmeters have been used as respiration flowmeters, but, in measuring the respiration at a low flow velocity of a newborn, for example, the respiration flowmeters of these types work unstably or more adversely can not measure the respiration flow rate. Generally, on the other hand, when comparing the expiration with the inspiration, there are great differences in temperature, humidity and gas composition. Therefore, even in case of the respiration flowmeter using the ultrasonic wave, a measuring error arising from a change of an ultrasonic propagating velocity in the respiration gas under measurement is not negligible. Thus, the conventional respiration flowmeters have been unsuccessfully in providing perfect solutions to the above problems.

Accordingly, an object of the present invention is to provide a respiration flowmeter which does not need a complex calculation circuitry and which can accurately measure the flow velocity, not influenced by the sonic velocity and without inflicting a pain or load on patients.

To achieve the above object, the respiration flowmeter as defined above is characterized in that the digital counting means is composed of a first clock means generating first clock pulses a second clock means generating second clock pulses having a frequency lower than that of the first clock, a first clock counting means for measuring the time difference ΔT by counting the

first clock pulses, and a second clock counting means for measuring the propagating period T1 by counting the second clock pulses, and that the calculating and storing means performs the calculation of $(2T1-\Delta T)^2$ for obtaining a value of $(T1+T2)^2$ using the measured values $\Delta T$ and T1.

This invention can be more fully understood from the following detailed description when taken in conjunction with the accompanying drawings, in which:

Fig. 1 shows a cross sectional view of a conduit provided with a pair of ultrasonic transducers used in an embodiment of a respiration flowmeter according to the present invention;

Fig. 2 is a block diagram illustrating an overall arrangement of the embodiment used together with the conduit shown in Fig. 1;

Fig. 3 shows timing diagrams of signals at the respective portions of the embodiment shown in Fig. 2; and

Figs. 4 and 5 illustrate flow charts useful in explaining the operation of the embodiment shown in Fig. 2.

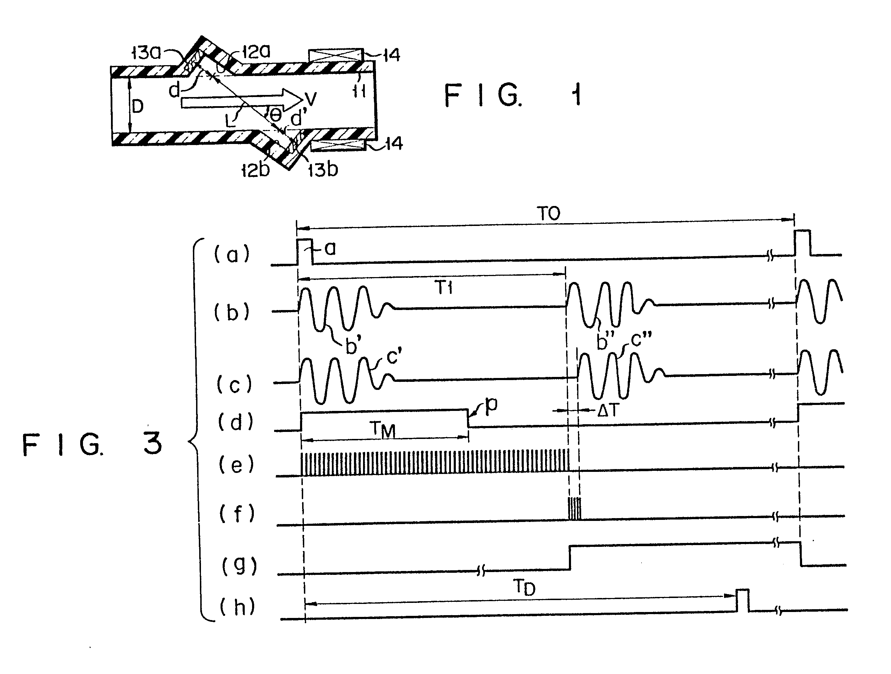

A structure of a conduit provided with a pair of ultrasonic transducers used in an embodiment of a respiration flowmeter according to the present invention will first be described referring to Fig. 1. In the figure, an expiration gas, for example, flows through a conduit 11, which is made of polycarbonate, for example, and has an inner diameter D, along its axis and at a flow velocity V in the direction of an arrow. The conduit 11 may also be made of metal or plastic. Accordingly, the inspiration gas flows at a flow velocity V' in the opposite direction to that of the arrow. A pair of recesses 12a and 12b are provided on the inner wall of the conduit 11 along a line slanted at a center of the respiration gas flow, for example, with respect to the gas flow. A pair of ultrasonic transducers 13a and 13b are respectively provided on the inner wall of the recesses 12a and 12b, facing at their transmitting and receiving surfaces each other along the slant line. Lead wires (not shown) for leading drive signals to the ultrasonic transducers 13a and 13b and ultrasonic signals received from the ultrasonic transducers 13a and 13b may be connected through the walls of the recesses 12a and 12b to the transducers 13a and 13b from the rear sides of the transducers, respectively. This structure allows the respiration gas to smoothly flow through the conduit 11 without being interrupted by the pair of transducers 13a and 13b. It is evident that, with this structure, a propagating velocity of the ultrasonic wave propagating portions d and d' within the recesses 12a and 12b are not influenced by a flow velocity of the respiration gas. Accordingly, a distance over which the propagating velocity is influenced by the flow velocity of the gas between the transducers 13a and 13b is L. If it is assumed that the portions d and d' are equal (d=d'), an angle of the direction V of the respiration gas flow with respect to the propagating direciton of the ultrasonic wave is θ and that a sonic velocity in the respiration gas is C, a propagating time T1 of the ultrasonic pulses radiated from the transducer 13a to the transducer 13b in the respiration flow direction V is given by

$$T1=\frac{2d}{C}+\frac{L}{C+V\cos\theta} \qquad (1)$$

An opposite propagating time T2 of the ultrasonic pulses radiated from the transducer 13b to 13a is

$$T2=\frac{2d}{C}+\frac{L}{C-V\cos\theta} \qquad (2)$$

A difference time $\Delta T$ between the propagating times T1 and T2 is

$$\Delta T=T1-T2=\frac{-2LV\cos\theta}{C^2-V^2\cos^2\theta}\fallingdotseq\frac{-2LV\cos\theta}{C^2} \qquad (3)$$
$$(\because V\ll C)$$

Rearranging the equation (3) with respect to the flow velocity V, we have

$$V=\frac{-C^2}{2L\cos\theta}\Delta T \qquad (4)$$

The sonic velocity C changes depending on temperature, humidity and gas composition of the respiration air. Accordingly, if the flow velocity of the respiration is measured by merely using the ultrasonic propagating time difference $\Delta T$, the measured value is influenced by the above factors.

Let us calculate the sonic velocity C by using the sum Ta of the two ultrasonic propagating times T1 and T2. Then,

$$Ta=T1+T2=2T1-\Delta T=\frac{4d}{C}+\frac{2CL}{C^2-V^2\cos^2\theta}.$$
$$\fallingdotseq\frac{2(2d+L)}{C} \qquad (5)$$
$$(V\ll C)$$

Rearranging the equation (5) with respect to the sonic velocity C, we obtain

$$C=\frac{2(2d+L)}{Ta}\fallingdotseq\frac{2(2d+L)}{2T1-\Delta T} \qquad (6)$$

Substituting the equation (6) into the equation (4), then we have

$$V=\frac{-2(2d+L)^2}{L\cos\theta}\cdot\frac{\Delta T}{Ta^2}=\frac{-2(2d+L)^2}{L\cos\theta}\cdot\frac{\Delta T}{(2T1-\Delta T)^2} \qquad (7)$$

The equation (7) implies that the flow velocity V can be measured by measuring only the ultrasonic propagating time difference $\Delta T$ and the ultrasonic propagating time T1, independently of the sonic velocity C, that is, without directly accounting for effects by temperature, humidity and gas composition.

In the structure shown in Fig. 1, an electric heater 14 is mounted around the conduit 11 and/or the transducers 13a and 13b, if necessary, in order to prevent the condensation made when the expiration of 100% humidity flows through the conduit 11.

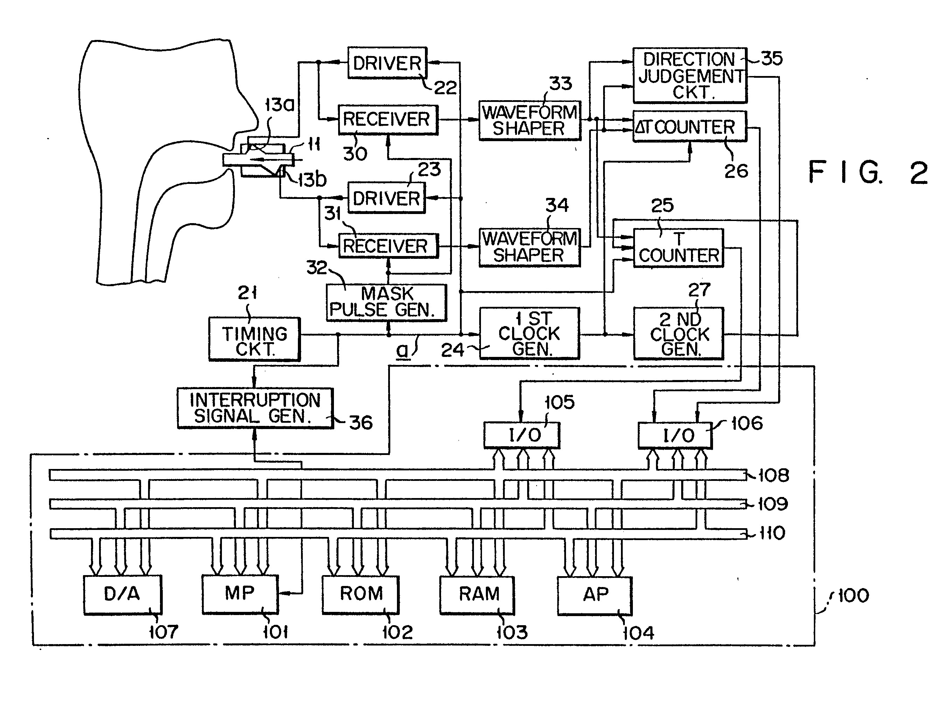

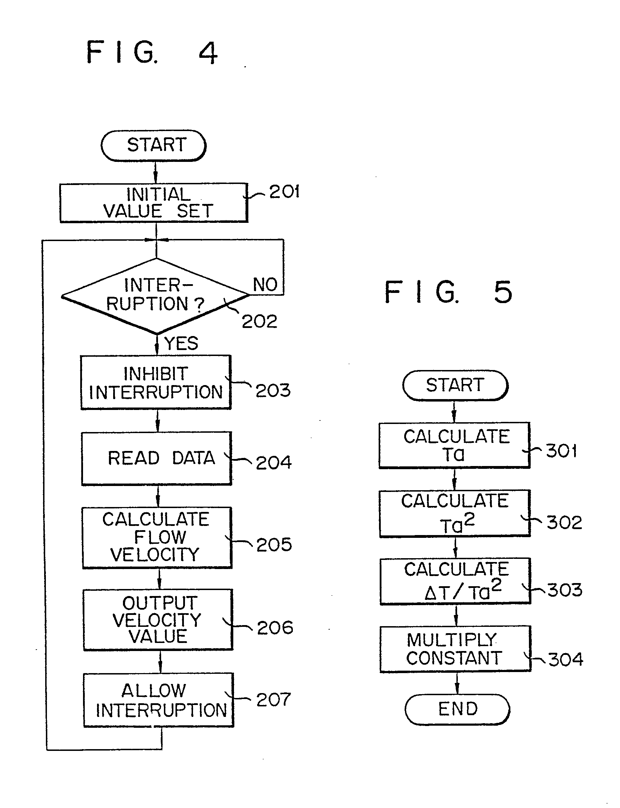

An arrangement of an embodiment of the present inventoin will be described referring to a block diagram of a device shown in Fig. 2 and signal waveforms shown in Fig. 3. In Fig. 2, a pulse a (Fig. 3(a)) produced from a timing circuit 21 for each time T0 is applied to driving circuits 22 and 23, a first clock generating circuit 24 and a T counter 25. The time T0 designates a time in the order of several msec to 10 msec. The first clock generating circuit 24 generates clock signals at 100 MHz, for example, for transfer to a $\Delta T$ counter 26 for counting the ultrasonic propagating time difference $\Delta T$ (0 to several μsec). The T counter 25 counts a propagating time T1, and starts to count in response to the pulse a. The clock signal used for counting the propagating time T1 is supplied from a second clock generating circuit 27. The second clock generating circuit 27 is driven by an output signal from the first clock generating circuit 24, and produces second clock signals at 10 MHz, which is, for example, 1/10 that of the first clock signals.

Upon receipt of the pulse a, the driving circuits 22 and 23 generate signals b' and c' (Figs. 3(b) and 3(c)) for driving ultrasonic transducers 13a and 13b. Ultrasonic waves simultaneously radiated from the ultrasonic transducers 13a and 13b travel toward the opposite side ultrasonic transducers 13b and 13a, and velocity of the waves is changed on the way by the flow velocity of the respiration gas flowing through the conduit 11. The ultrasonic waves transmitted are received by the corresponding transducers 13a and 13b and are transformed into electrical siganls b" and c" which are in turn applied to the receivers 30 and 31, respectively. Receivers 30 and 31, which are controlled by an output of a mask pulse generating circuit 32, reject signals applied during a period $T_M$ after the ultrasonic transducers 13a and 13b are driven (Fig. 3(d)). Accordingly, the receivers 30 and 31 discriminate signals b' and c' (Figs. 3(b) and 3(c)) for driving the ultrasonic transducers 13a and 13b from received signals b" and c", and receive only the received signals b" and c". The mask pulse generating circuit 32 is also driven by the output pulses from the timing circuit 21 and produces pulses d (Fig. 3(d)). The output signals from the receivers 30 and 31 are respectively applied to waveform shapers 33 and 34 where those are waveform-shaped, and those waveform-shaped ones are applied to a direction judgment circuit 35, the $\Delta T$ counter 26, and the T counter 25.

The T counter 25 receives the output signal from the waveform shaper 33 to terminate its counting which has been performed under drive of the timing circuit 21. The T counter 25 counts a time taken for an ultrasonic wave to travel from the ultrasonic wave transducer 13a to the transducer 13b (Fig. 3(e)). In the present embodiment, an inspiration condition is illustrated as shown in Fig. 2 in which a patient 10 breathes in the direction of the arrow. Under this condition, the signal b" goes ahead of the signal c", as shown in Fig. 3. In an expiration condition that the patient 10 breathes out, the signal c" goes ahead the signal b".

The $\Delta T$ counter 26 starts its counting responsive to the leading signal of those output signals from the wavefrom shapers 33 and 34, that is, the output signal from the waveform shaper 33 in this case, and stops its counting responsive to the lagged signal, that is, the output signal from the waveform shaper 34. In this way, the counter 26 counts the ultrasonic propagating time difference $\Delta T$, as shown in Fig. 3(f).

The direction judging circuit 35 also receives output signals from the waveform shapers 33 and 34. The circuit 35 judges that the respiration is the inspiration when the output signal from the waveform shaper 33 leads ahead the output signal derived from the waveform shaper 34. On the other hand, it judges that the respiration is the expiration when the former lags behind the latter. At this judgment, its output goes high (see Fig 3(g)) or low in level. The direction judging circuit 35 may be formed of a general flip-flop with a clear terminal.

At the time that the three data necessary for measuring the flow velocity, i.e. the direction of the respiration, the ultrasonic propagating time T1 and the ultrasonic propagating time difference $\Delta T$, are measured, an interruption signal which is the output pulse a derived from the timing circuit 21 delayed by time $T_D$, 1 msec, for example, is applied from an interruption signal generating circuit 36 (see Fig. 3(h)). The interruption signal is a signal for driving an arithmetic circuit 100 so as to cause it to calculate a flow velocity by using the above three data.

The arithmetic circuit 100 is comprised of a microprocessor (MP) 101, a read only memory (ROM) 102 for storing a program executed by the arithmetic circuit 100 and constants, a random access memory (RAM) 103 for storing the data produced during, before or after the execution of the program, an arithmetic processor (AP) 104 for executing the operations such as numerical operation, and ports (I/O) 105 and 106 for inputting the three data, and a digital to analog (D/A) converter 107 for producing a calculated flow velocity in the form of an analog signal. Those components are interconnected by a control bus 108, an address bus 109 and a data bus 110.

The arithmetic circuit 100 proceeds with the operation in accordance with a flow chart shown in Fig. 4.

In a step 201, following the power on, initial values are set in the microprocessor 101, the ports 105 and 106 for inputting data, the memory 102 and the like in the arithmetic circuit 100. Then, in a step 202, the arithmetic circuit 100 is in a standby mode for waiting the interruption signal. When the interruption pulse enters the microprocessor 101, the microprocessor 101 sets itself to be in an interruption inhibiting mode, in a step 203. In the next step 204, the microprocessor 101 fetches the data of the direction of the gas flow, the ultrasonic propagating time difference $\Delta T$, and the ultrasonic propagating time T1 from the ports 105 and 106 for the data inputting, arranges the data into a format easy to be processed. The data are then stored in the RAM 102. For ease of explanation, the $\Delta T$ will be treated as data with a sign representing a direction of the gas flow. Then, in a step 205, the microprocessor 101 executes a flow velocity of the gas in accordance with a flow chart shown in Fig. 5 by using the data in the step 204. The resultant data of flow velocity is delivered from D/A converter 107 through the step 206. Finally, in a step 207, the microprocessor 101 sets itself to be in an interruption permission mode and waits an interruption signal produced in the next transmission and reception of the ultrasonic wave. Through the repetition of the above-mentioned operation, the respiration flowmeter of the present embodiment can measure at a rate of about 100 times per second, a flow velocity and a direction of the pulsating gas flow which instantaneously change with the respiration.

The flow chart shown in Fig. 5 illustrating a process flow of the calculation of the flow velocity according to the equation (7) will be given hereinafter.

In a step 301, the ultrasonic propagating time T1 and the ultrasonic propagating time difference $\Delta T$ stored in the RAM 103 are set in the arithmetic processor (AP) 104 where the ultrasonic propagating time sum TA $(=2T1-\Delta T)$ is obtained. The $\Delta T$ used when the Ta is calculated has a sign representing the flow direction, as described above, and a direction of the inspiration flow is represented by a negative sign in the present embodiment.

In a step 302, an instruction for squaring the ultrasonic propagating time sum Ta is transferred to the AP 104 where $Ta^2$ is calculated. In the next step 303, the $\Delta T$ data is read out from the RAM 103 and set in the AP 104 where the ratio $\Delta T/Ta^2$ is calculated. Finally, in a step 304, the constant in the equation (7),

$$\frac{-2(2d+L)^2}{Lcos\theta}$$

is read out from the ROM 102 and is similarly set in the AP 104. Then, the MP 101 transfers an instruction to multiply the constant in the equation (7) by the ratio $\Delta T/Ta^2$ to the AP 104 where the flow rate is calculated. During the course of the operation, the data transmission between the ROM (102), the RAM (103) or the ports I/O 105 and 106 for data inputting and the microprocessor MP 101 is performed in a usual way. For example, the ROM 102 is designated through the address bus 109; a control signal for data read or data write is transferred to the ROM 102 through the control bus 108; the data to be read or written is transferred through the data bus 110.

As described above, the present invention successfully solves the problems of the prior art as previously stated and provides an almost ideal respiration flowmeter. It should be noted that, in the respiration flowmeter according to the present invention, the signal processing for the measurement is digitally performed, not through the D/A converting process, in the almost entire circuit portions except the ultrasonic transmitting and receiving portions. This feature eliminates the temperature drift which is problematic in the low flow rate measurement. Additionally, there is no need of the fine circuit adjustment essential to the analog circuit. This feature makes easy the manufacture of the flowmeter. Further, the analog to digital converter connected to the data processing is of course unnecessary, leading to simplification of the circuit construction, cost reduction and high reliability of the operation.

Further, the respiration flowmeter of the present invention, which measures the flow velocity of the respiration by using the ultrasonic propagating time and the ultrasonic propagating time difference, allows the frequency of the clock signal to be selected for securing a measuring accuracy required for the measurement to be made. Therefore, the circuit construction is simple and an unnecessary high speed circuit is omitted. Further, the values thus measured are arranged in a minimum data length, so that the succeeding data process may be effectively performed.

In the above-mentioned embodiment, the receiver circuits 30 and 31 are so connected to be supplied with the mask pulse for discriminate signals b' and c' for driving ultrasonic transducers 13a and 13b from received signals b" and c". The waveform shapers 33 and 34 may also be connected to be controlled by the mask pulse.

**Claims**

1. A respiration flowmeter comprising:
a conduit (11) through which expiration and inspiration flow, the conduit (11) having a pair of recesses formed respectively on opposite portions of the inner wall of the conduit (11) along an axis inclined to the direction of respiration gas flow;
a pair of ultrasonic transducers (13a, 13b) disposed in the recesses, respectively, and having ultrasonic transmitting and receiving surfaces facing each other and perpendicular to the axis;

switching means (22, 23, 30—32) for simultaneously switching the operation modes of said ultrasonic transducers (13a, 13b), from a drive mode to a receiving mode, and vice versa;

digital counting means (25, 26) for receiving signals generated by the transducers upon reception of ultrasonic pulses and measuring the two propagating periods required by ultrasonic pulses radiated from each of said driven transducers (13a, 13b) to reach the receiving transducers and the difference between these propagating periods;

judging means (35) for judging the direction of the respiration gas flow;

means (36) for generating a signal for requesting calculation of a flow velocity of the respiration gas every time said transducers (13a, 13b) are driven;

storing means (103) for storing signals representing said flow direction, the propagation periods, and said propagation period difference, in response to said calculation request signal; and

means (100) for calculating and storing the flow rate of said respiration gas on the basis of the values stored in said storing means (103), said calculating and storing means (100) including an arithmetic processor (104) for obtaining the flow velocity V by executing the following operation:

$$V = \frac{-2(2d+L)^2}{L \cos \theta} \cdot \frac{\Delta T}{(T1+T2)^2}$$

where d is the length of the ultrasonic transmission path in one of said recesses free from the effect of the flow velocity of said gas, L is the length of the ultrasonic transmission path in which the flow velocity of said gas is finite, θ is the angle between the respiration flow direction and the axis, T1 is the ultrasonic propagating period of the ultrasonic pulses radiated from one to the other of the pair of ultrasonic transducers, T2 is the ultrasonic propagating period of the ultrasonic pulses radiated from the other to the one of the pair of the ultrasonic transducers, and ΔT is the time difference between the two propagating periods T1 and T2, characterized in that said digital counting means (24 to 27) is composed of a first clock means (24) generating first clock pulses, a second clock means (27) generating second clock pulses, having a frequency lower than that of the first clock, a first clock counting means (26) for measuring said time difference ΔT by counting the first clock pulses, and a second clock counting means (25) for measuring the propagating period T1 by counting the second clock pulses, and that said calculating and storing means (100) performs the calculation of $(2T1-\Delta T)^2$ for obtaining a value of $(T1+T2)^2$ using said measured values ΔT and T1.

2. A respiration flowmeter according to claim 1, characterized in that an electric heater (14) for preventing condensation is provided on the periphery of said conduit (11) and/or around the ultrasonic transducers (13a, 13b).

3. A respiration flowmeter according to claim 1, characterized in that said switching means (22, 23, 30—32) include a circuit (32) which supplies a mask pulse for preventing receiving circuits (30, 31) or waveform shapers (33, 34) from receiving signals when drive circuits (22, 23) are driven.

**Patentansprüche**

1. Atemstrom-Meßgerät, umfassend

eine Leitung (11), durch die Ausatem- und Einatemluft strömt und die zwei jeweils in gegenüberliegenden Abschnitten der Innenwand der Leitung (11) längs einer zur Richtung des Atemgasstroms schräggestellten Achse ausgebildete Vertiefungen aufweist,

zwei in den jeweiligen Vertiefungen angeordnet Ultraschall-Wandler (13a, 13b) mit einander zugewandten und senkrecht zur genannten Achse stehenden Ultraschall-Sende- und -Empfangsflächen,

Schalt(er)einrichtungen (22, 23, 30—32) zum gleichzeitigen Umschalten der Betriebsarten der Ultraschall-Wandler (13a, 13b) von einer Treiberbetriebsart auf eine Empfangsbetriebsart und umgekehrt,

eine digitale Zähleinrichtung (25, 26) zum Empfangen der von den Wandlern bei Empfang von Ultraschallimpulsen erzeugten Signale und zum Messen der beiden Ausbreitungszeiten, welche die von jedem der angesteuerten Wandler (13a, 13b) abgestrahlten Ultraschallimpulse bis zum Erreichen der empfangenden Wandler benötigen, sowie der Differenz zwischen diesen Ausbreitungszeiten,

eine Entscheidungseinrichtung (35) zur Bestimmung der Richtung des Atemgasstroms,

eine Einrichtung (36) zum Erzeugen eines Signals für Anforderung einer Berechnung der Strömungsgeschwindigkeit des Atemgases bei jedesmaliger Ansteuerung der Wandler (13a, 13b),

eine Speichereinrichtung (103) zum Speichern von Signalen entsprechend der Strömungsrichtung, den Ausbreitungszeiten und der Ausbreitungszeitdifferenz nach Maßgabe des Berechnungs-Anforderungssignals, und

eine Einrichtung (100) zum Berechnen und Speichern der Strömungs- oder Durchsatzmenge des Atemgases auf der Grundlage der in der Speichereinrichtung (103) gespiecherten Werte oder Größen,

wobei die Rechen- und Speichereinrichtung (100) einen arithmetischen Prozessor (104) zur Ableitung der Strömungsgeschwindigkeit V durch Ausführung folgender Operation aufweist:

$$V = \frac{-2(2d+L)^2}{L \cos \theta} \cdot \frac{\Delta T}{(T1+T2)^2}$$

worin bedeuten: d=die Länge der Ultraschall-Sendestrecke in einer der Vertiefungen, ohne den Einfluß der Strömungsgeschwindigkeit des Gases, L=Länge der Ultraschall-Sendestrecke, in welcher die Strömungsgeschwindigkeit des Gases endlich ist, θ=Winkel zwischen der Atemstromrichtung und der Achse, T1=Ultraschall-Ausbreitungszeit der vom einen zum anderen der beiden Ultraschall-Wandler ausgestrahlten Ultraschallimpulse, T2=Ultraschall-Ausbreitungszeit der vom anderen zum einen der beiden Ultraschall-Wandler ausgestrahlten Ultraschallimpulse und ΔT=Zeitdifferenz zwischen den beiden Ausbreitungszeiten T1 und T2, dadurch gekennzeichnet,

daß die digitale Zähleinrichtung (24—27) aus einer ersten Takteinheit (24) zur Erzeugung erster Taktimpulse, einer zweiten Takteinheit (27) zur Erzeugung zweiter Taktimpulse einer Frequenz, die niedriger ist als diejenige der ersten Taktimpulse, einer ersten Taktzähleinheit (26) zum Messen der Zeitdifferenz ΔT durch Zählen der ersten Taktimpulse sowie einer zweiten Taktzähleinheit (25) zum Messen der Ausbreitungszeit T1 durch Zählen der zweiten Taktimpulse aufgebaut ist und

daß die Rechen- und Speichereinrichtung (100) die Berechnung von (2T1−ΔT)² zur Ableitung einer Größe von (T1+T2)² unter Heranziehung der Meßgrößen ΔT und T1 ausführt.

2. Atemstrom-Meßgerät nach Anspruch 1, dadurch gekennzeichnet, daß ein elektrisches Heizelement (14) zur Verhinderung einer Kondensation am Umfang der Leitung (11) und/oder um die Ultraschall-Wandler (13a, 13b) herum angeordnet ist.

3. Atemstrom-Meßgerät nach Anspruch 1, dadurch gekennzeichnet, daß die Schalt(er)einrichtungen (22, 23, 30—32) eine Schaltung (32) aufweisen, die einen Maskierungsimpuls liefert, um Empfangsschaltungen (30, 31) oder Wellenformeinheiten (33, 34) am Empfang von Signalen zu hindern, wenn Treiberschaltungen (22, 23) angesteuert sind.

**Revendications**

1. Débimètre respiratoire comprenant:
une conduite (11) dans laquelle l'expiration et l'inspiration passent, la conduite (11) possédant une paire d'évidements respectivement formés de part et d'autre de la paroi interne de la conduite (11) suivant un axe incliné par rapport à la direction d'écoulement du gaz respiratoire;
une paire de transducteurs d'ultrasons (13a, 13b) respectivement disposés dans les évidements et possédant des surfaces d'émission et de réception d'ultrasons situées en regard l'une de l'autre et perpendiculairement à l'axe;
des moyens de commutation (22, 23, 30 à 32) servant à faire simultanément commuter les modes de fonctionnement des transducteurs d'ultrasons (13a, 13b), d'un mode de commande à un mode de réception, et inversement;

des moyens de comptage numérique (25, 26) servant à recevoir des signaux produits par les transducteurs dès la réception d'impulsions ultrasonores et à mesurer les deux durées de propagation nécessaires pour que des impulsions ultrasonores rayonnées par chacun desdits transducteurs commandés (13a, 13b) atteignent les transducteurs récepteurs, et la différence entre ces durées de propagation;
des moyens de décision (35) servant à déterminer le sens de l'écoulement du gaz respiratoire;
des moyens (36) servant à produire un signal permettant de demander, le calcul de la vitesse d'écoulement du gaz respiratoire à chaque fois que lesdits transducteurs (13a, 13b) sont commandés;
des moyens d'emmagasinage (103) servant à emmangasiner des signaux représentant ledit sens d'écoulement, les durées de propagation, et ladite différence des durées de propagation, en réponse audit signal de demande de calcul; et
des moyens (100) permettant de calculer et d'emmagasiner le débit dudit gaz respiratoire à partir des valeurs emmagasinées dands lesdits moyens d'emmagasinage (103),
lesdits moyens de calcul et d'emmagasinage (100) comportant un processeur arithmétique (103) permettant d'obtenir la vitesse d'écoulement V en exécutant l'opération suivante:

$$V = \frac{-2(2d+L)^2}{L\cos\theta} \cdot \frac{\Delta T}{(T1+T2)^2}$$

où d est la longueur du trajet d'émission des ultrasons dans l'un desdits évidements dégagé de l'effet de la vitesse d'écoulement dudit gaz, L est la longueur du trajet d'émission des ultrasons dans lequel la vitesse d'écoulement dudit gaz est finie, θ est l'angle entre le sens d'écoulement de la respiration et l'axe, T1 est la durée de propagation des impulsions ultrasonores rayonnées de l'une à l'autre transducteur de la paire de transducteurs d'ultrasons, T2 est la durée de propagation des impulsions ultrasonores rayonnées de l'autre au premier transducteur de la paire de transducteurs d'ultrasons, et ΔT est la différence entre les deux durées de propagation T1 et T2, caractérisé en ce que:
lesdits moyens de comptage numérique (24 à 27) sont constitués par une première horloge (24) produisant de premières impulsions d'horloge, une deuxième horloge (27) produisant des deuxièmes impulsions d'horloge à une fréquence plus basse que celle de la première horloge, un premier moyen (26) de comptage des signaux d'horloge servant à mesurer ladite différence ΔT par comptage des premières impulsions d'horloge, et un deuxième moyen (25) de comptage d'impulsions d'horloge servant à mesurer la durée de propagation T1 par comptage des deuxièmes impulsions d'horloge, et
lesdits moyens de calcul et d'emmagasinage (100) effectuent le calcul de (2T1−ΔT)² permettant d'obtenir une valeur de $(T1+T2)^2$ à l'aide desdites valeurs $\Delta T$ et $T1$ mesurées.

2. Débimètre respiratoire selon la revendication 1, caractérisé en ce qu'un élément chauffant électrique (14) destiné à empêcher la condensation est placé sur la périphérie de ladite conduite (11) et, ou bien, autour des transducteurs d'ultrasons (13a, 13b).

3. Débimètre respiratoire selon la revendication 1, caractérisé en ce que lesdits moyens de commutation (22, 23, 30 à 32) comportent un circuit (32) qui délivre une impulsion de masquage destinée à empêcher que des circuits de réception (30, 31) ou des conformateurs d'onde (33, 34) ne reçoivent de signaux lorsque les circuits de commande (22, 23) sont commandés.

F I G. 1

F I G. 3

0 051 293

F I G. 2

0 051 293

# F I G. 4

```
        ( START )
            │
   ┌─────────────────┐
   │    INITIAL      │ ─── 201
   │  VALUE  SET     │
   └─────────────────┘
            │
            ▼
        ╱─────────╲          NO
       ╱  INTER-   ╲─────────
       ╲ RUPTION ? ╱
        ╲─────────╱  202
            │ YES
            ▼
   ┌─────────────────┐
   │    INHIBIT      │ ─── 203
   │  INTERRUPTION   │
   └─────────────────┘
            │
            ▼
   ┌─────────────────┐
   │   READ  DATA    │ ─── 204
   └─────────────────┘
            │
            ▼
   ┌─────────────────┐
   │   CALCULATE     │
   │     FLOW        │ ─── 205
   │   VELOCITY      │
   └─────────────────┘
            │
            ▼
   ┌─────────────────┐
   │    OUTPUT       │
   │   VELOCITY      │ ─── 206
   │    VALUE        │
   └─────────────────┘
            │
            ▼
   ┌─────────────────┐
   │     ALLOW       │ ─── 207
   │  INTERRUPTION   │
   └─────────────────┘
```

# F I G. 5

```
        ( START )
            │
            ▼
   ┌─────────────────┐
   │   CALCULATE     │ ─── 301
   │      Ta         │
   └─────────────────┘
            │
            ▼
   ┌─────────────────┐
   │   CALCULATE     │ ─── 302
   │      Ta²        │
   └─────────────────┘
            │
            ▼
   ┌─────────────────┐
   │   CALCULATE     │ ─── 303
   │    ΔT / Ta²     │
   └─────────────────┘
            │
            ▼
   ┌─────────────────┐
   │    MULTIPLY     │ ─── 304
   │   CONSTANT      │
   └─────────────────┘
            │
            ▼
        (  END  )
```